# EUROPÄISCHE PATENTANMELDUNG

(11) **EP 4 119 080 A1**
(43) Veröffentlichungstag der Anmeldung: **18.01.2023**
(21) Anmeldenummer: 21184980.7
(22) Anmeldetag: 12.07.2021
(51) Int. Cl.: A61B 18/04, A61B 18/14

(54) **PLASMASONDE UND VERFAHREN ZUR MONTAGE IHRER ELEKTRODE**

(71) Anmelder: Erbe Elektromedizin GmbH, 72072 Tübingen (DE)
(72) Erfinder: Heym, Johannes, 72070 Tübingen (DE)
(74) Vertreter: Rüger Abel Patentanwälte PartGmbB

(57) **Zusammenfassung**

Eine erfindungsgemäße Plasmasonde (11) weist einen Schlauch mit einem darin angeordneten Leiter auf, der zumindest an seinem distalen Ende eine Elektrode (20) trägt. Die Elektrode (20) ist entweder direkt auf den Leiter (21) gesichert oder der Leiter (21) ist zumindest an seinem distalen Ende mit einer Kunststoffummantelung versehen, von der die Elektrode (20) gehalten ist. Die Elektrode (20) kann zwischen dem Leiter (21) und die Kunststoffummantelung (22) eingeschoben und dadurch eingeklemmt sein. Nach Erstinbetriebnahme kann die Kunststoffummantelung (22) mit der Elektrode (20) verschmolzen sein. In jedem Fall liegt der Leiter (21) jedoch mit Spiel in einem Kanal oder einem Hohlraum der Elektrode (20), wodurch auch bei punktueller Berührung zwischen dem Leiter (21) und der Elektrode (20) infolge des sonst zwischen beiden vorhandenen Spalts die Wärmeübertragung von der Elektrode (20) auf den Leiter (21) behindert und somit der Wärmeeintrag in die Plasmasonde (11) begrenzt wird. Dies kommt der Lebensdauer der Plasmasonde (11) zugute und senkt zugleich deren Außentemperatur und damit deren Klebeneigung am Gewebe.

## Beschreibung

Gegenstand der Erfindung ist eine Plasmasonde, insbesondere eine Argon-Plasmasonde, sowie ein Verfahren zur Montage einer Elektrode dieser Plasmasonde.

Elektrochirurgische Instrumente mit Elektroden, die direkt auf ein Gewebe einwirken, sind bekannt. Beispielsweise offenbart die WO 2017/076721 A1 ein Hochfrequenzwerkzeug mit einer als Resektionsschlinge ausgebildeten Elektrode. Die U-förmig gebogene Elektrode steckt mit ihren beiden Enden in entsprechenden Aufnahmen, zu denen jeweils ein elektrischer Zuleitungsdraht führt. Die Elektrode ist mit diesem Zuleitungsdraht vercrimpt oder verschweißt, jedenfalls aber mechanisch fest verbunden. Eine Leitungsisolierung der elektrischen Zuleitung erstreckt sich dabei über das Ende der Elektrode und isoliert dieses gegenüber einer äußeren Umhüllung.

Aus der EP 1 568 325 A1 ist ein Instrument zur Eileiterkoagulation bekannt, das an seinem distalen Ende einen mit mehreren Elektroden versehenen Aufsatz aufweist. Diese sind über einen Koaxialstecker mit einer Koaxialzuleitung verbunden. Diese aus Zuleitung und Stecker bestehende Anordnung kann im Arbeitskanal eines Endoskops axial frei bewegt werden.

Des Weiteren existieren Plasmasonden, die im Wesentlichen aus einem an eine Gasquelle anschließbaren Schlauch bestehen, dessen distales Ende offen ist und zur Abgabe eines Plasmastrahls dient. In dem distalen Ende des Schlauchs ist eine Elektrode angeordnet, die typischerweise mittels eines Elektrodenhalters, beispielsweise eines sich diametral durch das Lumen des Schlauchs erstreckendes Metallplättchens, gehalten ist. Die Elektrode setzt sich als Leitungsdraht in proximaler Richtung durch das Lumen des Schlauchs fort und ist an eine elektrische Quelle anschließbar, um am distalen Ende eine elektrische Entladung hervorzurufen. Nach diesem Prinzip aufgebaute Plasmasonden sind außerdem aus der DE 10 2017 127 976 A1, der DE 100 30 111 B4 oder der EP 3 769 707 A1 bekannt.

Von der Elektrode der Plasmasonde geht ein Plasmastrom aus, der das zu behandelnde Gewebe treffen soll. Weil die Elektrode in dem distalen Ende des Schlauchs angeordnet ist, gelangt aber mindestens ein Teil der erzeugten Wärmeenergie auch auf die umgebende Wandung des distalen Endes des Schlauchs. Zum Schutz desselben kann hier eine Keramikhülse angeordnet werden, wie es beispielsweise aus der oben genannten WO 2005/046495 A1 bekannt ist. Die zentrische Lagerung der Elektroden wird jedoch von dem Metallplättchen übernommen, das Wärme auf den Schlauch überträgt und bei längerer Einsatzdauer zu dessen Zerstörung führen kann. Dies insbesondere, weil die Elektrode fest mit dem Metallplättchen verbunden sein muss, sodass ein erheblicher Wärmeübergang von der Elektrode auf das Plättchen gegeben ist. Diese Problematik stellt sich erst recht, wenn das Metallplättchen selbst als Elektrode dient wie zum Beispiel in der DE 100 30 111 B4 vorgeschlagen.

Hiervon ausgehend, ist es Aufgabe der Erfindung, ein Konzept anzugeben, mit dem sich eine langzeitstabile Plasmasonde auf einfache Weise bereitstellen lässt. Dabei soll besonderes Augenmerk auf die Montage der Elektrode gelegt werden.

Diese Aufgabe wird mit der Plasmasonde nach Anspruch 1 sowie mit dem Verfahren nach Anspruch 15 gelöst:

Die erfindungsgemäße Plasmasonde weist einen zum Beispiel aus einem Kunststoffmaterial bestehenden flexiblen Schlauch auf, der ein Lumen oder mehrere Lumina aufweisen kann, die sich jeweils von seinem proximalen Ende bis zu seinem distalen Ende erstrecken. Das Lumen dient dazu, ein geeignetes Gas, vorzugsweise ein Inertgas, beispielsweise Argon, von dem proximalen Ende zu dem distalen Ende zu leiten. Das Lumen des Schlauchs ist an dem distalen Ende offen, sodass das Gas hier ausströmen kann.

In dem Schlauch ist ein elektrischer Leiter angeordnet, der sich vorzugsweise über die gesamte Länge des Schlauchs von dem proximalen Ende bis zu dem distalen Ende desselben erstreckt und dort an eine Elektrode angeschlossen ist. Der elektrische Leiter ist zumindest an seinem distalen Ende mit einer Kunststoffummantelung versehen. Die Kunststoffummantelung kann aus dem gleichen Kunststoff bestehen wie der Schlauch. Es ist aber auch möglich, unterschiedliche Kunststoffe vorzusehen.

Die Elektrode ist vorzugsweise eine gerade ausgebildete nadel- oder stabartige Elektrode, die ein proximales und ein distales Ende aufweist. Die Elektrode ist insgesamt oder wenigstens über einen Teil ihrer Länge hohl ausgebildet, d.h. sie weist einen inneren, an beiden Enden offenen oder am distalen Ende geschlossenen Kanal auf, der einen Hohlraum bildet. Der Leiter erstreckt sich mit seinem distalen Ende mit Spiel in diesen Hohlraum hinein. Die Elektrode wird dadurch von dem Leiter und, zumindest bei einigen Ausführungsformen der Erfindung, auch von der Kunststoffummantelung des Leiters getragen. Es ist möglich, die Elektrode so anzuordnen, dass sich ihr proximales Ende in die Kunststoffummantelung des Leiters erstreckt und von dieser axial fixiert ist. In ungebrauchtem Zustand kann die Fixierung durch Reibschluss zwischen der Kunststoffummantelung und der Elektrode erfolgen. Nach erstem Einsatz kann die Kunststoffummantelung an die Elektrode angeschmolzen und dadurch mit dieser verklebt sein, sodass eine stoffschlüssige Verbindung zwischen dem proximalen Ende der Elektrode und der Kunststoffummantelung gegeben ist.

Vorzugsweise erstreckt sich der Leiter mit Spielpassung in die Elektrode. Durch die immer gegebene leichte Welligkeit oder Biegung des Leiters oder auch nur aufgrund der nicht vollständigen Fluchtung zwischen dem Leiter und der Elektrode ist immer ein elektrischer Kontakt zwischen dem Leiter und der Elektrode gegeben. Außerdem ist die an dem Leiter anliegende HF-Spannung so hoch, dass ein etwaiger Spalt zwischen dem Leiter und der Elektrode vom Strom ohne weiteres überwunden wird. Nach erstem Einsatz kann es an Kontaktstellen zwischen dem Leiter und der Elektrode zu einem geringfügigem Materialfluss kommen, sodass sich eine stoffschlüssige Verbindung ähnlich einer Löt- oder Schweißverbindung ausbildet.

Unabhängig davon, ob sich die Elektrode in die Kunststoffummantelung hinein erstreckt oder nicht, kann die Elektrode zur Fixierung auf den Leiter etwas radial nach innen zu verformt sein, beispielsweise durch Quetschen oder zu Crimpen. Eine solche Verformung kann am distalen Ende der Elektrode, am proximalen Ende der Elektrode oder dazwischen ausgebildet sein. Der Leiter erstreckt sich dann in distaler Richtung wenigstens bis über den verformten Bereich hinaus.

Eine nach diesem Konzept aufgebaute Plasmasonde lässt sich einfach herstellen. Die Herstellung umfasst folgende Schritte: - Bereitstellung eines Schlauchs, der ein proximales Ende und ein distales Ende aufweist, zwischen denen wenigstens ein Lumen ausgebildet ist, wobei einem in dem Schlauch ein elektrischer Leiter angeordnet ist, der sich von dem proximalen Ende des Schlauchs bis zu dessen distalen Ende erstreckt, der eine Kunststoffummantelung aufweist, - optional Entfernen der Kunststoffummantelung von einem distalen Abschnitt des Leiter, so dass dieser frei liegt, - Bereitstellen einer Elektrode, die wenigstens ein hohl ausgebildetes Ende aufweist, - Aufschieben des hohlen Endes der Elektrode auf das vorzugsweise von der Kunststoffummantelung befreite Ende des Leiters und, Einschieben des hohlen Endes der Elektrode in die Kunststoffummantelung des Leiters. Die Elektrode kann somit in einem einzigen Montageschritt in die Plasmasonde eingesetzt und darin fixiert werden, indem sie lediglich auf das vorzugsweise freiliegende Ende des Leiters aufgesteckt und gegebenenfalls in den zwischen dem Leiter und der Kunststoffummantelung sich öffnenden Zwischenraum eingeschoben wird. Durch das dabei erfolgende Aufweiten der Kunststoffummantelung liegt diese unter Vorspannung fest an der Elektrode an.

Die Elektrode besteht vorzugsweise aus einem Metall. Sie kann insbesondere an ihrer Außenseite mit einer Beschichtung versehen sein, insbesondere einer Beschichtung aus einem Metall oder einer Metall-Legierung. Vorzugsweise weist das Metall der Beschichtung eine Schmelztemperatur auf, die niedriger ist, als die Schmelztemperatur des Materials, aus dem die Elektrode besteht. Als Beschichtungsmetall eignen sich Silber oder Silberlegierungen. Es können aber auch andere Metalle, insbesondere Metalle mit niedriger Oxdidationsneigung und/oder hoher elektrischer und/oder thermischer Leitfähigkeit zur Anwendung kommen.

Ein besonderer Vorzug der erfindungsgemäßen Struktur liegt in der thermischen Entkopplung der Elektrode von dem Schlauch. Auch wenn die Elektrode nach längerem Einsatz heiß wird, kann sie den äußeren Schlauch nicht infolge von Wärmeleitung aufschmelzen. Die erfindungsgemäße Plasmasonde weist somit ein gegenüber herkömmlichen Sonden kälteres distales Ende auf, eine höhere Standzeit der Sonde ermöglicht. Auch wird die Beeinflussung von Gewebe durch von der seonde ausgehende Wärme gemindert. Zudem wird an der flächigen Verbindungsstelle zwischen dem proximalen Ende der Elektrode und der Kunststoffummantelung des Leiters beim Einsatz eine geringfügige Aufschmelzung der Kunststoffummantelung und dadurch eine feste stoffschlüssige Verbindung zwischen der Elektrode und dem Leiter bzw. seiner Kunststoffummantelung hergestellt.

Weiter gestattet das erfindungsgemäße Konzept eine exakte sowohl axiale als auch radiale Ausrichtung der Elektrode. Selbst wenn der Schlauch kurzzeitig äußerlich verformt wird, wird dadurch die Positionierung der Elektrode nicht beeinträchtigt.

Das von dem Schlauch umschlossene Lumen kann in zwei oder mehrere zueinander parallel verlaufende Teillumina unterteilt sein. Die Unterteilung kann sich über die gesamte Länge des Schlauchs oder lediglich über eine Teillänge erstrecken. Die Unterteilung des Lumens in Teillumina kann durch radial ausgerichtete oder schräg zur Radialrichtung ausgerichtete Wände erfolgen, die den Schlauch mit der Kunststoffummantelung verbinden. Die Wände können dazu beitragen, den Leiter in dem Schlauch zentral zu lagern und zu halten. Der Schlauch kann somit mitsamt der Kunststoffummantelung des Leiters und der mindestens einen Verbindungswand zwischen dem Schlauch und der Kunststoffummantelung in einem Arbeitsgang, zum Beispiel durch Kunststoffextrusion, hergestellt werden. Vorzugsweise wird dabei nur ein Kunststoffmaterial verwendet. Es ist aber auch möglich, für den Schlauch und die Kunststoffummantelung unterschiedliche Kunststoffe zu verwenden und die Sonde durch Koextrusion zu erzeugen. Es ist zudem auch möglich, die Kunststoffummantelung und den Schlauch als voneinander unabhängige Elemente aus gleichen oder unterschiedlichen Kunststoffmaterialien bereitzustellen. Der Leiter mit seiner Kunststoffummantelung kann in dem Schlauch beweglich, beispielsweise in Axialrichtung und/oder in Radialrichtung beweglich angeordnet sein.

Bei allen diesen Ausführungsformen existiert keine metallische Verbindung zwischen dem Leiter oder der Elektrode einerseits und dem Schlauch andererseits. Falls eine stoffliche Verbindung zwischen der Kunststoffummantelung und dem Schlauch existiert, ist diese Verbindung vorzugsweise frei von Metallelementen oder anderen gut wärmeleitenden Elementen. Auf diese Weise wird die Wärmeleitung zwischen dem der Elektrode und dem Schlauch minimiert. Ergänzend kann das distale Ende des Schlauchs mit einer vorzugsweise elektrisch isolierenden, beispielsweise aus Keramik bestehenden temperaturfesten Hülse versehen sein, um einen direkten Kontakt zwischen dem sich ausbildenden Plasmastrom und dem aus Kunststoff bestehend Schlauch zu vermeiden.

Die lediglich an ihrem proximalen Ende gehaltene Elektrode erstreckt sich vorzugsweise freitragend von dem Leiter in distaler Richtung weg, ohne aus dem Lumen des Schlauchs heraus zu ragen. So kann ein direkter Kontakt zwischen der Elektrode und biologischem Gewebe verhindert werden. Es ist jedoch auch möglich, die Elektrode aus dem Schlauch heraus ragend anzuordnen, wobei dann vorzugsweise auf dem distalen Ende der Elektrode ein Isolatorkörper platziert ist.

Weitere Eigenschaften und Merkmale der erfindungsgemäßen Plasmasonde lassen sich der Zeichnung oder der nachfolgenden Beschreibung entnehmen. Es zeigen.
Figur 1 eine erfindungsgemäße Plasmasonde angeschlossen an ein speisendes Gerät, in schematischer Darstellung,
Figur 2 das distale Ende der Plasmasonde, in schematisierter perspektivischer Darstellung,
Figur 3 eine Stirnansicht der Plasmasonde nach Figur 2
Figur 4 die Plasmasonde nach Figur 3, geschnitten entlang der in Figur 3 eingetragenen strichpunktierten Linie IV-IV,
Figur 5 eine abgewandelte Ausführungsform der erfindungsgemäßen Plasmasonde, in längsgeschnittener Darstellung,
Figur 6 bis 8 weiter abgewandelte Ausführungsformen der erfindungsgemäßen Plasmasonde, jeweils in längsgeschnittener Darstellung,
Figur 9 eine Plasmasonde mit Isolatorkörper in teilweise aufgeschnittener Seitenansicht,
Figur 10 eine Stirnansicht einer abgewandelten Ausführungsform der erfindungsgemäßen Plasmasonde und
Figur 11 eine Stirnansicht einer weiteren abgewandelten Ausführungsform der erfindungsgemäßen Plasmasonde.

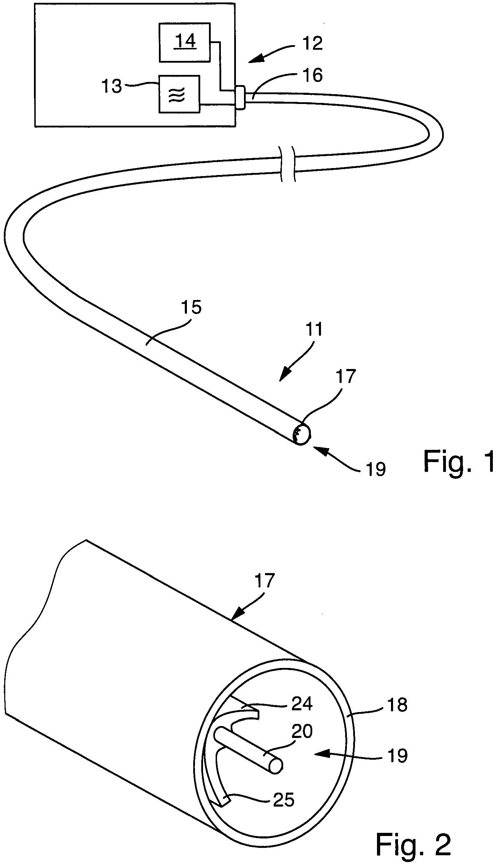

In Figur 1 ist eine Plasmasonde 11 veranschaulicht, die an ein speisendes Gerät 12 angeschlossen ist. Das Gerät 12 stellt die zum Betrieb der Plasmasonde 11 nötigen Betriebsmedien und elektrische Leistung bereit. Dazu enthält das Gerät 12 einen Hochfrequenzgenerator 13 sowie eine Gasquelle 14. Diese umfasst beispielsweise Druckregler und Ventile, über die ein aus einer Gasflasche entnommenen Gasstrom, beispielsweise Argonstrom, kontrolliert zu der Plasmasonde 11 geleitet werden kann.

Die Plasmasonde 11 weist einen Schlauch 15 auf, der sich von einem proximalen Ende 16 bis zu einem distalen Ende 17 erstreckt. Die Stirnfläche 18 des distalen Endes 17 de Schlauchs 15 umgibt eine Plasma-Ausströmöffnung 19, die in Betrieb einen Plasmastrahl abgibt. Mit den Begriffen Ende, distales Ende und proximales Ende werden immer Endbereiche in Bezug genommen.

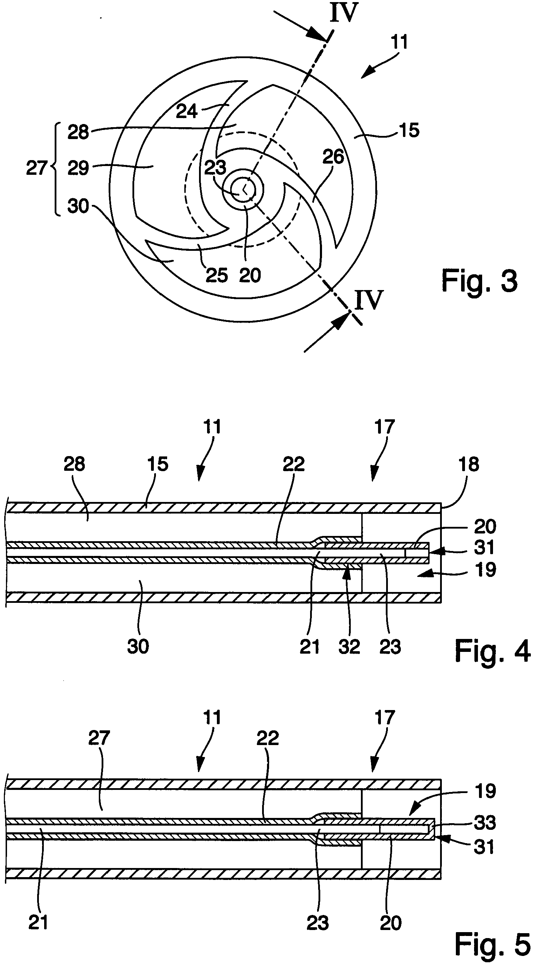

In der Plasma-Ausströmöffnung 19 ist außerdem eine Elektrode 20 angeordnet, die elektrisch mit dem HF-Generator 13 verbunden ist. Dazu dient ein beispielsweise aus Figur 3 und 4 ersichtlicher Leiter 21, der sich durch die gesamte Länge des Schlauchs 15 von seinem proximalen Ende 16 zu seinem distalen Ende 17 erstreckt. Die Elektrode 20 kann bei allen Ausführungsformen aus einem temperaturfesten Material, z.B. Edelstahl bestehen. Außerdem kann sie bei allen Ausführungsformen mit einer Beschichtung, insbesondere einer Beschichtung versehen sein, deren Schmelztemperatur vorzugsweise niedriger ist, als die Schmelztemperatur der Elektrode 20. Insbesondere kann die Beschichtung aus Silber oder einer Silberlegierung bestehen.

Der Leiter 21 kann beispielsweise durch einen monofilen Draht beispielsweise einen Edelstahldraht oder auch durch einen aus einem anderen Material bestehenden Draht gebildet sein. Der Leiter 21 ist dabei zumindest über einen Teil seiner Länge mit einer Kunststoffummantelung 22 versehen, die den Leiter 21 vorzugsweise an seinem gesamten Umfang (360°) umgibt. Die Kunststoffummantelung 22 kann sich dabei über die gesamte Länge des Leiters bis zu seinem distalen Ende hin 23 hin erstrecken. Das distale Ende 23 des Leiters 21 kann selbst freiliegen, d.h. frei von Kunststoffummantelung sein. Der freiliegende Abschnitt kann ein oder mehrere Millimeter lang sein. Die Kunststoffummantelung 22 erstreckt sich, ausgehend von dem distalen Ende 23, wenigstens einige Zentimeter in proximaler Richtung. Sie kann aber auch die gesamte Länge des Leiters 21 einnehmen.

Bei dem in Figur 4 veranschaulichten Ausführungsbeispiel ist die Kunststoffummantelung 22 über mindestens eine, vorzugsweise mehrere Wände 24, 25, 26 mit dem Schlauch 15 verbunden wie aus Figur 3 hervorgeht. Diese unterteilen ein von dem Schlauch 15 umschlossenes Lumen 27, in zwei oder mehrere, hier drei, Teillumina 28, 29, 30. Die Wände 24, 25, 26 können, wie in Figur 3 ersichtlich, gegen die Radialrichtung geneigt bspw. in Spiralrichtung verlaufen oder auch anderweitig angeordnet sein. Außerdem können die Wände sowohl eben als auch wie dargestellt gekrümmt ausgebildet sein.

Die Elektrode 20 kann, wie in Figur 4 veranschaulicht ist, durch ein Metallröhrchen gebildet sein. Dieses weist einen zentralen Kanal oder Hohlraum auf, in den sich das distale Ende 23 des Leiters 21 hinein erstreckt. Vorzugsweise ist dabei der Innendurchmesser dieses Kanals oder Hohlraums etwas größer als der Außendurchmesser des Leiters 21, sodass sich zwischen beiden eine Spielpassung ergibt. Im Ausführungsbeispiel nach Figur 4 ist die Elektrode 20 hohlzylindrisch ausgebildet und an ihrem distalen Ende 31 offen. Das proximale Ende 32 der Elektrode 20 ist soweit auf den Leiter 21 aufgeschoben, dass es sich zwischen die Kunststoffummantelung 22 und den Leiter 21 geschoben hat. Figur 4 veranschaulicht dabei, dass die Kunststoffummantelung 22 lokal von dem Leiter 21 gelöst und geweitet ist, wobei sie die Elektrode 20 dadurch zunächst mindestens reibschlüssig sichert. Das distale Ende 23 des Leiters 21 liegt lose in dem Kanal bzw. Hohlraum der Elektrode 20 und dabei zunächst punktuell an der Elektrode 20 an. Vorzugsweise ist die Verbindung zwischen dem Leiter 21 und der Elektrode 20 in Axialrichtung lose, d.h. nicht zugkraftübertragend.

Bei allen vor- und nachstehend beschriebenen Ausführungsformen mit hülsenförmiger Elektrode 20 kann diese an ihrem distalen Ende eine schräg zu ihrer Längsrichtung orientierte Endfläche aufweise. Die röhrchen-förmige Elektrode 20 kann dazu wie das distale Ende der Kanüle einer Spritze an ihrem distalen Ende schräg zu ihrer Achse gekappt sein.

Unabhängig von der Schrägstellung der Endfläche der Elektrode 20 kann sich die Zuleitung durch die Elektrode 20 hindurch erstrecken und über das distale Ende der Elektrode 20 hinaus stehen. Dies kann zur Verbesserung der Zündwilligkeit beitragen.

Die insoweit beschriebene Plasmasonde 11 kann hergestellt werden, indem zunächst der Schlauch 15 mit dem darin angeordneten Leiter 21 bereitgestellt wird. Beispielsweise kann der Schlauch 15 mit dem Leiter 21 wie ein Kabel durch Kunststoffextrusion erzeugt werden. Von dem so bereitgestellten Material wird die für die Plasmasonde 11 gewünschte Länge abgeschnitten und der Leiter 21 an seinem distalen Ende 23 zunächst freigelegt. Dabei wird das entsprechende Material der Kunststoffummantelung 22 und der Wände 24 bis 26 entfernt. Damit liegt das distale Ende 23 des Leiters 21 frei.

In einem nachfolgenden Arbeitsschritt wird nun die Elektrode 20 auf das freiliegende distale Ende 23 des Leiters 21 aufgeschoben und in die Kunststoffummantelung 22 eingeschoben. Wie sich aus Figur 4 erkennen lässt, drängt die Elektrode 20 dabei die Kunststoffummantelung 22 radial nach außen und wird dadurch selbst eingeklemmt. Die Elektrode 20 ist nun reibschlüssig gehalten. Das distale Ende 23 des Leiters 21 liegt punktuell an der inneren Wandung der Elektrode 20 lose an. Vorzugsweise wird die Elektrode 20 dabei so weit eingeschoben, dass sie von außen gesehen, hinter der distalen Stirnfläche 18 des Schlauchs 15 steht, d.h. proximal gegen diese Stirnfläche 18 versetzt ist. Die Plasmasonde 11 ist nun einsetzbar.

Zum Betrieb der Plasmasonde 11 wird diese an das Gerät 12 angeschlossen. Dabei wird das proximale Ende des Leiters 21 elektrisch mit dem HF-Generator 13 verbunden. Das proximale Ende des Lumens 27 wird mit der Gasquelle 14 verbunden. Zum Einsatz wird das Lumen 27 mit Gas, beispielsweise Argon, oder einem anderen Inertgas versorgt, sodass in dem Lumen 27 ein in distaler Richtung fließender Gasstrom entsteht. Der HF-Generator 13 versorgt nun die Elektroden 20 mit HF-Spannung von typischerweise mehreren 100 Volt gegenüber einem Neutralpotential, das über eine nicht weiter dargestellte Neutralelektrode an den zu behandelnden Patienten angelegt ist.

An der Elektrode 20 entsteht nun ein sogenannter Funken, mit dem das ausströmende Gas ionisiert wird, sodass sich ein Plasmastrahl bildet. Der Strom fließt dabei von dem Leiter 21 über die Berührungspunkte zwischen den distalen Ende 23 und der Elektrode 20 auf die Elektrode 20 und von dieser über das ionisierte Gas zu dem Patienten. Der Stromfluss kann dabei ein punktuelles Verlöten oder Verschweißen des Leiters 21 mit der Elektrode 20 und somit eine mechanische Verbindung bewirken. Außerdem heizt sich die Elektrode 20 erheblich auf, wodurch die Kunststoffummantelung 22 in dem die Elektrode 20 überdeckenden Bereich schmelzen oder anschmelzen kann. Damit bildet sich eine stoffschlüssige Verbindung zwischen der Ummantelung 22 und der Elektrode 20 und/oder zwischen dem Leiter 21 und der Elektrode 20.

An der Plasmasonde 11 können Abwandlungen vorgenommen werden, ohne den Bereich der Erfindung zu verlassen. Beispielsweise kann gemäß Figur 5 auf jede der Wände 24, 25, 26 verzichtet werden. Der Leiter 21 liegt mit seiner Ummantelung 22 dabei lose in dem Lumen 27 und kann darin axial und/oder radial bewegt werden.

Unabhängig davon ist es möglich, die Elektrode 20 mit einem geschlossenen Ende 33 zu versehen, das den distalen Abschluss der Elektrode 20 bildet. Insbesondere hinsichtlich der Verbindung zwischen dem Leiter 21 und der Elektrode 20 gelten die obigen Ausführungen entsprechend.

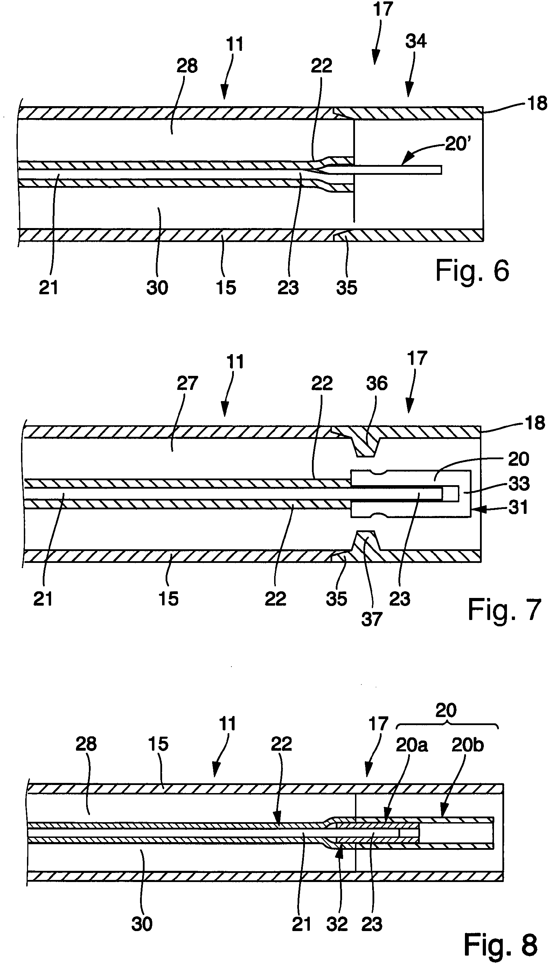

Auch hinsichtlich der Verbindung zwischen der Elektrode 20 und dem Leiter 21 sind zahlreiche Abwandlungen möglich. Zum Beispiel kann anstelle einer hülsenförmigen Elektrode 20 gemäß Figur 4 oder 5 auch eine nadel- oder stäbchenförmige Elektrode 20' Anwendung finden wie es in Figur 6 veranschaulicht ist. Auch diese kann zwischen den Leiter 21 und die Kunststoffummantelung 22 geschoben und dadurch eingeklemmt werden. Der Leiter 21 kann wie bei den vorbeschriebenen Ausführungsbeispielen ein massiver Draht sein. Es kann jedoch ersatzweise bei dieser Ausführungsform und auch bei den vorbeschriebenen Ausführungsformen nach Figur 3 und 4 anstelle eines massiven Drahts eine Litze Anwendung finden.

Bei der Plasmasonde nach Figur 6 kann bei der Herstellung auf eine Freilegung des distalen Endes 22 des Leiters 21, d.h. auf die Entfernung der Kunststoffummantelung 22 in diesem Bereich verzichtet werden. Während der Leiter 21 bzw. dessen distales Ende 23 bei den Ausführungsformen nach Figur 1 bis 4 die Elektrode 20 beim Einfügen in die Kunststoffummantelung 22 führt, ist eine derartige Führung bei der Ausführungsform nach Figur 6 nicht erforderlich. Die an ihrem proximalen Ende vorzugsweise angespitzte Elektrode 20' wird einfach in Nachbarschaft zu dem Leiter 21 in die Kunststoffummantelung 22 eingestochen.

Bei allen Sonden nach Figur 3 bis 6 kann das distale Ende 17 des Schlauchs 15 durch eine temperaturfeste Hülse 34 zum Beispiel aus Keramik gebildet sein. Dies ist in Figur 6 beispielhaft für alle anderen Ausführungsformen veranschaulicht. Die Hülse 34 kann über einen konischen Sitz 35 mit dem Schlauch 15 verbunden sein.

Es ist nicht zwingend erforderlich, die Elektrode 20 zwischen den Leiter 21 und dessen Kunststoffummantelung 22 zu schieben und durch Einklemmung zu fixieren. Figur 7 veranschaulicht dazu eine Ausführungsform der Plasmasonde 11, bei der die Elektrode 20 nur mit dem von der Kunststoffummantelung 22 befreiten distalen Ende 23 des Leiters 21 verbunden ist. Die Elektrode 20 kann durch radiale Deformation, beispielsweise Crimpen oder dergleichen, verliersicher auf dem Ende 23 des Leiters 21 gehalten sein. In Figur 7 ist rein beispielhaft eine Plasmasonde 11 beschrieben, bei der der Leiter 21 sowie dessen Kunststoffummantelung 22 und die Elektrode 20 nicht fest mit dem Schlauch 15 verbunden sind. Das Bauprinzip mit nur auf dem Leiter 21 gesicherter Elektrode 20 kann auch an jeder anderen der vorbeschriebenen Plasmasonden 11 verwirklicht sein. Außerdem können alle vor- und nachstehend beschriebenen Anordnungen aus Elektrode 20, Leiter 21 und Kunststoffummantelung 22 auch in Sonden zum Einsatz kommen, bei denen keine Verbindung zwischen dem Schlauch 15 und der Kunststoffummantelung 22 besteht. Z.B. kann der Leiter 21 mit der Kunststoffummantelung 22 als einadriges Kabel in den Schlauch 15 eingelegt sein.

Bei jeder Sonde, bei der der Leiter 21 und dessen Ummantelung 22 nicht mit dem Schlauch 15 verbunden sind, kann die am distalen Ende 17 angeordnete Hülse 34 drei oder mehrere nach innen weisende Nasen 36, 37 oder eine sonstige Struktur aufweisen, die die radiale Beweglichkeit der Elektrode 20 oder des Leiters 21 begrenzt. Die Nasen 36, 37 sind deswegen geeignet, eine ausreichende Zentrierung der Elektrode 20 zu bewirken. Sofern die Elektrode 20 mit dem Leiter 21 mechanisch verbunden ist, beispielsweise durch radiale Quetschungen wie in Figur 7 dargestellt, kann auf die Kunststoffummantelung 22 auch gänzlich verzichtet werden. Dies gilt für alle Ausführungsformen.

Figur 8 veranschaulicht eine weitere Abwandlung der Erfindung, die bei allen hier beschriebenen Plasmasonden 11 anwendbar ist. Die Elektrode 20 besteht aus einer ersten Hülse 20a, die auf dem distalen Ende 23 des Leiters 21 sitzt und in die Kunststoffummantelung 22 eingeschoben ist. Auf dieser Hülse 20a sitzt eine zweite Hülse 20b, die beispielsweise mit der Hülse 20a verschweißt oder vercrimpt ist oder einfach reibschlüssig auf dieser sitzt. Die beiden Hülsen 20a, 20b bestehen vorzugsweise aus unterschiedlichen Materialien oder Materialkombinationen. Beispielsweise kann die hohlzylindrische Hülse 20b an ihrer äußeren Fläche versilbert sein, wodurch die Plasmaentladung auf das distale Ende derselben konzentriert und der Wärmeeintrag in diese Hülse 20b minimiert wird. Die Hülse 20a kann hingegen aus unbeschichtetem Edelstahl mit schlechter Wärmeleitfähigkeit bestehen, sodass der Wärmeeintrag in die Kunststoffummantelung 22 minimiert wird. Unabhängig von der Materialwahl kann durch einen Abstand zwischen der Hülse 20b und der Kunststoffummantelung 22 der Wärmeeintrag in den Kunststoff vermindert werden.

Die Hülse 20a und die Fuge zwischen den Hülsen 20a und 20b stellt eine thermische Barriere zwischen dem der Entladung ausgesetzten Teil der Elektrode 20 und der übrigen Plasmasonde 11 dar. Dies erhöht die Standfestigkeit der Elektrode 20 und der gesamten Plasmasonde 11 zum einen aufgrund der Vergrößerung der Elektrodenoberfläche und zum anderen aufgrund der Reduktion des von der Elektrode 20 ausgehenden Wärmeflusses.

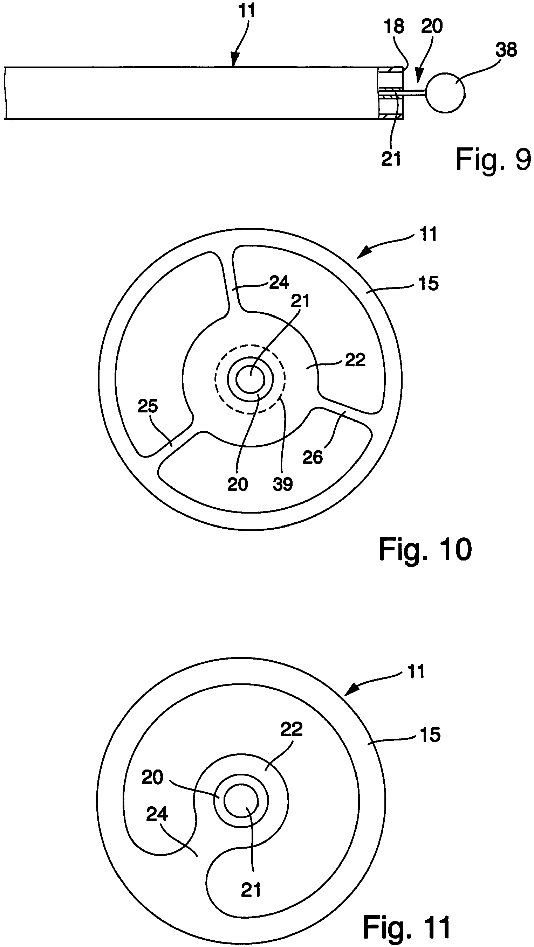

Bei allen vorbeschriebenen Ausführungsformen der Plasmasonde 11 wurde vorausgesetzt, dass die Elektrode 20, 20' die distale Stirnfläche 18 des Schlauchs 15 nicht überragt. Jedoch können auf jeder der vorbeschriebenen Ausführungsformen aufbauend auch Plasmasonden 11 nach dem Vorbild der Figur 9 bereitgestellt werden. Die Elektrode 20, die nach jeder der vorbeschriebenen Arten mit dem Leiter 21 verbunden sein kann, überragt dann die Stirnfläche 18 in distaler Richtung und kann einen Isolatorkörper 38 beispielsweise aus Keramik oder auch einem temperaturfesten Kunststoff tragen. Der Isolatorkörper 38 kann dabei kugelförmig, pilzförmig oder anderweitig geformt sein und wird von der Elektrode 20 getragen.

Hinsichtlich der Gestaltung des Schlauchs 15 und der Kunststoffumhüllung 22 bestehen zahlreiche Freiheiten. Zum Beispiel können die Wände 24, 25, 26, wie es Figur 10 zeigt, radial angeordnet werden. Auch kann der Leiter 21 zunächst von einer Isolierung 39 umgeben sein, die in die Kunststoffummantelung 22 eingebettet ist. Weiter kann die Anzahl der Wände oder sonstigen Verbindungen zwischen der Kunststoffummantelung 22 und dem Schlauch 15 von den vorbeschriebenen Ausführungsformen abweichend festgelegt sein wie Figur 11 zeigt. Dort ist lediglich eine einzige verbindende Wand 24 zwischen dem Schlauch 15 und der Kunststoffummantelung 22 vorgesehen.

Eine erfindungsgemäße Plasmasonde 11 weist einen Schlauch mit einem darin angeordneten Leiter auf, der zumindest an seinem distalen Ende eine Elektrode 20 trägt. Die Elektrode 20 ist entweder direkt auf den Leiter 21 gesichert oder der Leiter 21 ist zumindest an seinem distalen Ende mit einer Kunststoffummantelung 22 versehen, von der die Elektrode 20 gehalten ist. Die Elektrode 20 kann zwischen dem Leiter 21 und die Kunststoffummantelung 22 eingeschoben und dadurch eingeklemmt sein. Nach Erstinbetriebnahme kann die Kunststoffummantelung 22 mit der Elektrode 20 verschmolzen sein. In jedem Fall liegt der Leiter 21 jedoch mit Spiel in einem Kanal oder einem Hohlraum der Elektrode 20, wodurch auch bei punktueller Berührung zwischen dem Leiter 21 und der Elektrode 20 infolge des sonst zwischen beiden vorhandenen Spalts die Wärmeübertragung von der Elektrode 20 auf den Leiter 21 behindert und somit der Wärmeeintrag in die Plasmasonde 11 begrenzt wird. Dies kommt der Lebensdauer der Plasmasonde 11 zugute und senkt zugleich deren Außentemperatur und damit deren Klebeneigung am Gewebe. So sinkt die Gefahr der ungewollten Perforation von empfindlichen oder dünnen Gewebeschichten. Außerdem ermöglicht das erfindungsgemäße Konzept den langfristigen Erhalt der Rundheit der Sonde.

### Bezugszeichen:

- 11: Plasmasonde
- 12: Gerät
- 13: HF-Generator
- 14: Gasquelle
- 15: Schlauch
- 16: proximales Ende des Schlauchs 15
- 17: distales Ende des Schlauchs 15
- 18: distale Stirnfläche des Schlauchs 15
- 19: Plasma-Ausströmöffnung
- 20, 20': Elektrode
- 20a, 20b: Hülsen
- 21: Leiter
- 22: Kunststoffummantelung
- 23: distales Ende des Leiters
- 24 - 26: Wände
- 27: Lumen
- 28 - 30: Teillumina
- 31: distales Ende der Elektrode 20
- 32: proximales Ende der Elektrode 20
- 33: geschlossenes distales Ende der Elektrode 20
- 34: Hülse
- 35: konischer Sitz
- 36, 37: Nasen
- 38: Isolatorkörper

## Patentansprüche

1. Plasmasonde (11)
mit einem Schlauch (15), der ein proximales Ende (16) und ein distales Ende (17) aufweist, zwischen denen wenigstens ein Lumen (27) ausgebildet ist,
mit einem in dem Schlauch (15) angeordneten elektrischen Leiter (21), der sich von dem proximalen Ende (16) des Schlauchs (15) bis zu dessen distalen Ende (17) erstreckt und der eine Kunststoffummantelung (22) aufweist,
mit einer Elektrode (20), die ein mit dem Leiter (21) elektrisch verbundenes Ende (32) und ein sich in distaler Richtung erstreckendes Ende (33) aufweist,
wobei die Elektrode (20) von dem Leiter (21) und/oder der Kunststoffummantelung (22) des Leiters (21) gehalten ist.

2. Plasmasonde nach einem der vorstehenden Ansprüche, **dadurch gekennzeichnet, dass** das Lumen (27) an dem proximalen Ende (16) an eine Gasquelle (14) angeschlossen ist.

3. Plasmasonde nach einem der vorstehenden Ansprüche, **dadurch gekennzeichnet, dass** der Leiter (21) an seinem proximalen Ende an eine elektrische Quelle (13) angeschlossen ist.

4. Plasmasonde nach einem der vorstehenden Ansprüche, **dadurch gekennzeichnet, dass** das Lumen (27) in zwei oder mehrere zueinander parallel angeordnete Teillumina (28, 29) unterteilt ist.

5. Plasmasonde nach einem der vorstehenden Ansprüche, **dadurch gekennzeichnet, dass** der Leiter (21) in dem Schlauch (15) zentral angeordnet ist.

6. Plasmasonde nach einem der vorstehenden Ansprüche, **dadurch gekennzeichnet, dass** die Kunststoffummantelung (22) und der in dieser angeordnete Leiter (21) in dem Schlauch (15) beweglich angeordnet sind.

7. Plasmasonde nach einem der vorstehenden Ansprüche, **dadurch gekennzeichnet, dass** die Kunststoffummantelung (22) mit dem Schlauch (15) über wenigstens eine flexible Wand (24) oder über mehrere flexible Wände (24, 25, 26) miteinander verbunden sind.

8. Plasmasonde nach einem der vorstehenden Ansprüche 7 oder 8, **dadurch gekennzeichnet, dass** der Leiter (21) in dem Schlauch (15) ausschließlich mittels der flexiblen Wand (24) oder der flexiblen Wände (24, 25, 26) abgestützt ist.

9. Plasmasonde nach einem der vorstehenden Ansprüche, **dadurch gekennzeichnet, dass** das mit dem Leiter (21) verbundene Ende (32) der Elektrode (20) oder die Elektrode (20) insgesamt hohl ausgebildet ist und dass sich der Leiter (21) in die Elektrode (20) hinein und/oder durch diese hindurch erstreckt.

10. Plasmasonde nach einem der vorstehenden Ansprüche, **dadurch gekennzeichnet, dass** die Elektrode (20) eine Beschichtung aufweist.

11. Plasmasonde nach einem der vorstehenden Ansprüche, **dadurch gekennzeichnet, dass** sich das proximale Ende (32) der Elektrode (20) zwischen den Leiter (21) und die Ummantelung (22) erstreckt.

12. Plasmasonde nach einem der vorstehenden Ansprüche, **dadurch gekennzeichnet, dass** der Leiter (21) in der Elektrode (20) in Längsrichtung verschiebbar zu dieser angeordnet ist.

13. Plasmasonde nach einem der vorstehenden Ansprüche 1 bis 11, **dadurch gekennzeichnet, dass** die Elektrode (20) an dem Leiter (21) durch plastische Deformation gehalten ist.

14. Plasmasonde nach einem der vorstehenden Ansprüche, **dadurch gekennzeichnet, dass** das distale Ende (33) der Elektrode (20) mit einem Isolatorkörper (38) versehen ist.

15. Verfahren zur Montage einer Elektrode (20) einer Plasmasonde (11) mit folgenden Schritten:
Bereitstellung eines Schlauchs (15), der ein proximales Ende (16) und ein distales Ende (17) aufweist, zwischen denen wenigstens ein Lumen (27) ausgebildet ist, wobei einem in dem Schlauch (15) ein elektrischer Leiter (21) angeordnet ist, der sich von dem proximalen Ende (16) des Schlauchs (15) bis zu dessen distalen Ende (17) erstreckt, der eine Kunststoffummantelung (22) aufweist,
Bereitstellen einer Elektrode (20), die wenigstens ein hohl ausgebildetes Ende (32) aufweist,
Aufschieben des hohlen Endes (32) der Elektrode (20) auf das Ende (23) des Leiters (21) und,
Einschieben des hohlen Endes (32) der Elektrode (20) in die Kunststoffummantelung (22) des Leiters (21).
